# EUROPEAN PATENT APPLICATION

(11) **EP 4 119 751 A1**
(43) Date of publication of application: **18.01.2023**
(21) Application number: 21767831.7
(22) Date of filing: 12.03.2021
(51) Int. Cl.: E05B 47/00, E05B 59/00, E05C 1/02, G08B 13/06

(54) **INTERACTIVE SECURITY AND LOCKING SYSTEM FOR DOORS OF RESIDENCES**

(30) Priority: 12.03.2020 ES 202030468 U; 11.03.2021 ES 202130497 U
(71) Applicant: Perea Diaz, Roberto, 28022 Madrid (ES)
(72) Inventor: Perea Diaz, Roberto, 28022 Madrid (ES)
(86) International application number: PCT/ES2021/000011
(87) International publication number: WO 2021/180987

(57) **Abstract**

The invention relates to an interactive security and locking system for residential doors, linked to the floor (A), the door (B) and the electrical panel (C) of the dwelling, comprising mechanical means of physical locking, in particular at least one bolt (3), electrical and electronic security means for its opening and closing and means for its management through an application (App) for Android and IOS systems on Smartphone and Smartwatch mobile devices, said App connecting with the closure of security directly (locally) via Bluetooth and GSM and remotely (external server) via Wi-Fi, GPRS and LAN, also implementing means of controlling access to the home, means for connecting auxiliary peripheral elements of security and a security device for opening the door, acting as a self-contained system or as a peripheral unit.

## Description

### Object of the invention

The present application refers to an interactive security and locking system for dwellings doors that significantly improves the use of similar articles existing on the market and in the current state of the art.

Specifically, this innovation refers to a security system for dwellings doors with electronic activation and a system of anchor bolts on the floor that block the door, also adding means of activating optical and sound alerts. The main objective of this innovative closing mechanism is to increase the time that must be used to force the door, which is why it includes different remote access technologies and/or physical means of access that are complemented by means of connection for peripheral elements of security, control or warning.

### Technical area

This invention fits in the sector that corresponds to fixed constructions, in particular door or window locks and accessories, extensive to the operation of locks or other closing devices by electrical or magnetic means.

### Background of the invention and state of the art

One of the sectors that generates a greater volume of business is the security of dwellings, whether they are located in buildings or if they are single-family. The access doors are usually of a standard nature, made of wood. Some are completed with an "L" shaped bar located longitudinally and externally on the side of the door frame that is close to the lock. Obviously, placing protection bars is a good option on windows and interior doors leading to yards, terraces or balconies, but in no way can they be installed on the main access door to the dwelling.

At a technical level, different types of the classic key and bowler lock have been developed, from the cylindrical with a pear-shaped central cylinder where the key enters to the rim or mortise locks. In recent years, the so-called invisible locks have been developed, electronically operated and very easy to install, which lack a cylinder which, theoretically, is a serious drawback when trying to force it. But in general, the cylinder lock is the most used and is, statistically, the most vulnerable point of the door, so forcing said cylinder is the most used method for theft.

Regarding industrial property, there are numerous patents that develop locks and closing systems. This is the case of file ES1074663U, referring to a *"security device for doors" that "consists of a pair of metal tubes telescopically coupled to each other, arranged on the inner face of the door, crossing it from side to side; with the particularity that the tube with the largest diameter is articulated at its free end on an anchor provided in the wall, in correspondence with the hinged area of the door itself in which it is applied, while the free end of the tube with the smallest diameter is attachable in a housing established for this purpose in a plate provided in correspondence with the wall, on the opposite side to the hinge of the door itself, having foreseen that said tube of smaller diameter is associated with a bushing, movable along the same, provided with blocking means with respect to the aforementioned tube of smaller diameter, and provided in turn with anchoring means articulated to the door in the area opposite to the hinged area of the same."* European patent EP0963498A1 develops a *"lock assembly, which is arranged to be mounted on a hinged door, and a door opening activating device, which is arranged to be fixed to a fixed member facing the door. hinged door in a closed state thereof, the lock comprising a frame arranged to be fixed on the outside against a first side of a tubular member or fixed to said door; at least one bolt, which is slidably mounted on the frame between a retracted position and a protruding position, and which is arranged to extend in its protruding position, in the closed state of the door, through said tubular member, outside of a second state thereof, within said door closing activating device; and an actuation mechanism to move said bolt from its retracted position to its protruding position and vice versa."*

However, all the locks described, even fulfilling their function as a means of closure, have a serious drawback: they are all located near the door frame, which must be understood to be, together with the bowler, the weakest part of the door, unless it is an armored door mounted on a steel frame, which is still anchored in standard construction partition walls, so it remains vulnerable with the right tools.

There are also alarm systems installed in high-rise dwellings that are activated when the entrance door is forced, sending a signal to the owner or to the company that installs and controls them, which acts according to the established protocol, but the truth is that the alarm goes off when the intruder is already inside the dwelling and a certain time elapses until the owner or security personnel appear.

The applicant is not aware of a security system for dwellings doors similar to the one detailed in this application in the current state of the art.

### Description of the invention

The object of the invention presented is an interactive security and locking system for dwellings doors that incorporates technical characteristics that advantageously improve its operation.

Specifically, said security system for dwellings doors comprises two parts: a first part consisting of a mechanical physical locking means and a second part consisting of electronic security means for opening and closing such physical lock.

Innovatively, the mechanical means of physical blocking comprises at least one hardened steel bolt, solid or hollow, the lower part of which is housed in a cavity made in the interior floor of the dwelling, coinciding with the lower part of the door, with the bolt reaching up to the wrought. When the bolt is hollow, it houses a second crazy-turning cylindrical bolt inside.

The electronic security means for opening and closing the door has a double function. First of all, they activate the upward and downward movement of the bolts in the path they carry out to block the door - when they go down - or to release it - when they go up - allowing it to open and close on its hinge system. Secondly, said electronic means control different audible and/or optical alarm systems in the event of any attempt to force the door, including an alert system connected to at least one mobile device carried by the tenant of the dwelling.

The actuation of the bolts is activated by a transmitter/receiver control via radio with an encrypted signal and/or an evolutionary code received by a main motor, which acts on the bolts to lower or raise them and the door to be locked or unlocked. As a safety measure, the system has a second, secondary motor, with the same technical characteristics as the main motor and which is activated if the main motor has any incident that prevents its correct operation. The activation of this secondary motor can be carried out via GSM by means of an SMS in anticipation of the receiver/transmitter via radio suffering any failure, or through a wireless switch parallel to the installation, which would only work in the event of a fault. the other systems.

For its part, the system has a double system of sensors: at least one vibration sensor that detects any knock or vibration on the door with the intention of forcing it, i.e. the system is activated by the intruder's intentionality when the door has not yet been forced, and at least one magnetic sensor that is activated by a forced opening of the door.

Both sensors are part of the alarm system of the claimed mechanism and whose function is to dissuade the person who wants to force the door and simultaneously warn the tenant of the dwelling that an attempt is being made at that very moment. force his door.

In this regard, when at least one of the sensors is activated, an internal siren of at least 90 decibels is immediately activated, startling the intruder and alerting the neighbours. Simultaneously and through GSM technology, the tenant of the dwelling is notified by SMS to the previously registered telephone numbers.

This notification by SMS allows the tenant to contact directly the system electronic by phone call, so that, by having an amplifier and loudspeaker, their voice is heard live and direct on the landing of the apartment, this being a fact completely unexpected for the intruder, who at that moment doubts if there is someone inside the apartment.

It is also possible to incorporate a light alert connected to the door and/or to each landing of the block of flats and a discreet camera that allows the tenant to see the intruder and, if a recording system is available, save the images of the intrusion.

The different electrical and electronic elements that regulate the operation of the described system are housed in a central control module connected by wiring to the box that contains the bolts, motors and vibration sensor.

In summary, in the basic operation of the system, the bolt receives the impact of the leverage, transmits it to the box in which it is integrated and from this to the screws that hold it, in turn, to the door, which is likely to be different materials (wood, sheet metal, etc.) that may not withstand the impact sufficiently.

To provide a better technical solution, the bolts and/or the box incorporate a damping means to mitigate the impact of said leverage on the screws that hold the box and on the door. Regarding the bolts, their damping element is an intermediate piece that makes them flexible, so that when they receive the force of the leverage, they do not transmit it to the box or the bolts. The same result is achieved by incorporating a shock-absorbing element in the box, which, upon receiving the impact of the leverage force from the bolt, absorbs it and does not transmit it to the bolts that hold it.

In a new embodiment, the bolt system is structured as a superimposed equipment in which the bolt, or in its case the bolts, continue to be integrated in its box together with its activation mechanism (motor) and the vibration sensor, but a recessed cassette or receptacle is installed under the floor in which a mechanism that comprises some moving parts related to each other is located, so that the first piece receives the impact of the bolt when it penetrates the recessed cassette or receptacle and finally acts on the last piece, of variable conformation, for example another bolt, a platform, etc. moving it up. This ensures that the impact of the leverage received by the bolt reaches the door cushioned.

In another embodiment, the box is embedded in the floor, housing the main bolt or bolts and their consequent activation mechanism (motor) and the vibration sensor, achieving the same mechanical result regardless of the internal operating details. In this case, the box invades the space occupied by the corner of the door and the bolts go up inside the dwelling, so that they are not in any way in contact with the door, being a clear blocking system. In a lever, forcing the door open, less than 1 cm away there are some bolts that act as a block, so that the door transmits the force to the bolts, and these to the recessed cassette or receptacle integrated in the floor. A manual actuator related to the bolt system is added to unlock the equipment and the bolts go down, thus being able to leave the dwelling in an emergency.

In another embodiment, the bolt system together with its activation mechanism (motor) and the vibration sensor integrated in its box is located in a housing made in the same door, which implies that, at a construction level, it is necessary to form such an accommodation. In this case, the bolt or bolts go down and are inserted into the recessed cassette or receptacle in the floor, without any mechanical activation.

For maximum effectiveness as a means of blocking, in other embodiments, the box that contains the bolt or bolts together with its activation mechanism (motor) and the vibration sensor is installed in the area opposite the door hinge using screws, either in the door frame, on the wall or on the floor. In these implementation options, the bolt or bolts always move until they are blocking the opening of the door, without touching it at any time, but instead remain millimeters away from it, in the area of the frame, at different levels of the height of the door, being feasible to install more than one box in the same door at different points, which multiplies its resistance to leverage.

An optimal combination is the one resulting from installing the box with its corresponding bolt or bolts, activation mechanism (motor) and vibration sensor, i.e. a non-recessed cassette but installed in the door frame or wall on the opposite side to the hinges, as in the embodiment described in the previous paragraph but located in the lower part, combined with the perforations made in the floor, already described in the first embodiment, with which an articulated arm is achieved that rotates 90 degrees towards the side of the door, causing the bolts to enter the floor, acting as a block. This embodiment supposes a clear improvement in the resistance to leverage because the door transmits the force received to the bolts and these directly to the floor, never to the box, which does not receive any damage and the screws are not affected.

Optionally, if spatially feasible, the central control module and the magnetic sensor are integrated in the same box that contains the bolts, activation mechanism (motor) and vibration sensor.

The exposed physical locking system, even with emergency means of manual opening and the alert means already exposed (SMS, etc.), has been designed to be linked and managed through an application (APP) that connects with the equipment either wirelessly, or wired, directly (locally) via Bluetooth and GSM and remotely (external server) via Wi-Fi, GPRS and LAN, suitable for Android and IOS systems in Smartphone and Smartwatches mobile devices, implementing physical devices linked to the opening and access control of the door, which are integrated into the central control module, and incorporating, either in the central control module, either in the box or simultaneously in both, a device optional security for opening the door in case of failure of the wireless system, keeping said box its original content, i.e. the bolts, the vibration sensor, the magnetic sensor, the limit switch and the lever that allows the upward movement of the bolts to be activated manually to open the door from inside the dwelling. This optional security device for opening the door in the event of a failure of the wireless system incorporated in the central control module is specified in a main electronic board and management elements.

This interactive system encompasses both means for controlling access to the dwelling and means for connecting a plurality of peripheral elements, among others, sensors, detectors and alarm signals.

The means of access control are devices that act as switches that activate and deactivate the equipment connected to the main electronic board housed in the central control module that governs the operation of the system.

Both the access control means and the peripheral elements are externally accessible and duly related to the corresponding electronics, interacting with the tenant in different ways according to their corresponding technical characteristics

Some are located in areas designed and suitable to accommodate, for example, these technical characteristics, the environment of the dwelling or the tenant's criteria.

Others are linked to a device carried by the tenant and that controls access to the dwelling through wireless communication technologies such as Bluetooth, GSM/GPRS, WIFI and RF (radio frequency), sending an input to which the main electronic board of the module responds. central control. It is understood that these wireless means also constitute a support device in case of failure or violation of the aforementioned external devices.

Access control devices include, among others, the following:
- Alphanumeric keyboard
- Magnetic flat key
- Tubular key lock
- Manual push button
- Manual IR infrared push button
- Biometric fingerprint sensor type reader.
- Biometric facial sensor type reader.
- Biometric of the palm fingerprint sensor type reader
- Biometric of the eye iris sensor type reader.
- Biometric Forearm vein sensor type reader.
- Voice identification device
- RF emitter (radio frequency)
- Technological modules of wireless technology integrated circuit suitable to be read by the mobile electronic device through an APP or smartwatch, in particular low frequency RFID tag and card reader (LF: 125 - 134 kHz and 140 - 148.5 kHz) and high frequency (HF: 13.56 MHz) and its corresponding reader, as well as a CQR code reader and NFC technology and a reader for it.
- SMS

The means for connecting peripheral elements allow various auxiliary safety devices to be powered from the same system that regulates the interactive closure, i.e. from the central control module and through the corresponding settings, which are connected in accordance with the needs of each dwelling.

These auxiliary safety devices include, among others, the following:
- optical and/or sound alert terminal
- access control camera
- IP camera
- Thermographic camera
- gas sensor
- humidity sensor
- smoke sensor
- motion sensor
- infrared motion sensor
- radar motion sensor
- vibration sensor
- magnetic sensor
- camera motion sensor
- microwave motion sensor
- thermal sensor
- Volumetric

Both the access control devices and the auxiliary security devices are integrated into the interactive system that is claimed according to the requirements of each installation and the security needs that must be covered, choosing, in each group, one or the other without limitation of their number and in the right combinations.

### Description of the drawings

For a better understanding of what is described in this report, some drawings are attached, which should be analyzed and considered solely as an example and without any limiting or restrictive character.
Figure 1.- Diagram of the security closing system for dwellings doors with the door closed
Figure 2.- Diagram of the security closing system for dwellings doors with the door open
Figure 3.- Detail of the upward movement of the bolts with manual actuation by means of a lever
Figure 4.- Front schematic view of the bolt system in an overlay embodiment
Figure 5.- Detailed schematic view of the bolt system in an embodiment of overlapping in inactive mode
Figure 6.- Detail schematic view of the bolt system in an embodiment of overlapping in active mode, blocking the door
Figure 7.- Front schematic view of the bolt system housed in a recessed box in the floor
Figure 8.- Front schematic view of the system of bolts housed in their recessed box in a housing made in the door
Figure 9.- Front schematic view of the system of locking bolts housed in their box located in the area opposite the door hinge
Figure 10.- Front schematic view of the bolt system in function of locking housed in at least two boxes located in the area opposite the door hinge
Figure 11.- Front schematic view of the bolt system with angular displacement movement in function inactive lock, housed in its box located in the area opposite the door hinge
Figure 12.- Front schematic view of the system of bolts with angular displacement movement as a function of active locking, housed in their box located on the side of the door opposite the hinge.
Figure 13.- Diagram of the interactive closure in a side view of the door
Figure 14.- Diagram of the interactive closure in a side view of the door

### Description of a preferred embodiment

These figures explicitly detail the security system for dwellings doors that is claimed in this application.

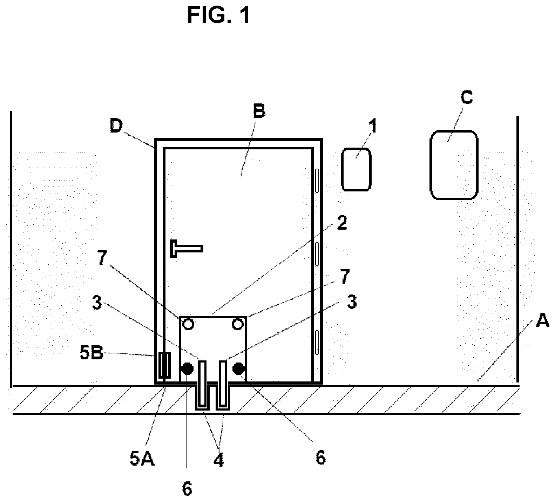

Figures 1 to 3 show the basic configuration of the invention, with the central control module related to the box containing the bolts, motors and vibration sensor.

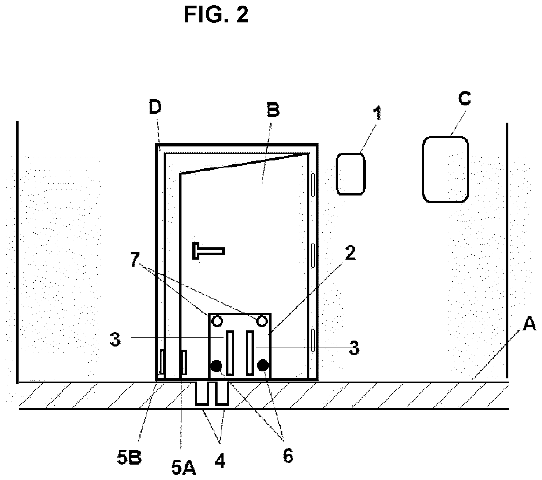

Figure 1 shows schematically the arrangement of the elements that make up this system related to the access door to the dwelling in the closed position, while figure 2 shows the door in the open position.

Both figures show an example an embodiment of the system, in which the floor (A), the door (B), its frame (D) and the electrical switchboard (C) of the dwelling. participate, although they are not claimed

In the rear part of the door (B), at a point close to its lower part, there is a box (2) inside which are housed some bolts (3), some main and secondary motors (7) and at least one vibration sensor (6), the box being connected by wiring (not drawn in these figures) with a central control module (1) containing electrical and electronic elements, this central control module (1) being located in an area close to the electrical switchboard (C) of the dwelling. In at least one point of the frame (D) and the door (B) a magnetic sensor is located, which consists of two pieces located in parallel and close to contact each other, a piece (5B) anchored in the frame (D) and the other (5A) on the door (B). In the floor (A) of the dwelling, in coincidence with the position of the bolts (3) housed in the box (2), two cavities (4) are made, suitable for housing the lower part of said bolts (3) when the door it is closed, as detailed in figure 1. The motors, both the main and the secondary, work at low voltage and the box (2) incorporates a backup battery in the event of an accidental or provoked power outage.

Figure 2 shows the door open, with the bolts (3) retracted or raised and located completely inside the box (2), while the two pieces of the magnetic sensor (5B) and (5A) are separated. The bolts (3) are mobile and carry out an ascending and descending path.

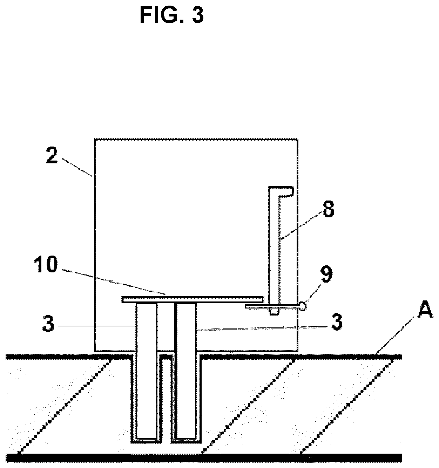

Figure 3 shows the detail of the upward movement of the manually operated bolts (3) in the case of total failure of the motors or the electronic system. The box (2) internally incorporates a rail (8) on which a lever (9) sits, which protrudes from the outside and which, when manually pushed upwards, moves the bolts (3) as a whole, all of them linked in their upper end to a longitudinal bar (10) that drags them up by the action of the already mentioned lever (9). The rail (8) regulates the travel lever (9). This outer lever does not appear in figures 1 and 2.

Based on these figures, the system operation is simple. When an intruder tries to force the door, he must necessarily come into physical contact with it, forcing the lock, prying etc. In any case, the vibration sensor (6) is activated, which is inside the box (2) close to the bolts (3), both anchored in the same support, with the bolts (3) in the locked position, i.e. inserted in the corresponding cavities (4) existing in the floor (A). If the forcing persists and the door lock is violated, the bolts (3) allow the door to move only between 5 mm to 15 mm, a very short distance with which the bolts (3) continue to have the door blocked. In this way, when the intruder acts on the door, the vibration sensor or sensors (6) are activated, connected to the central control module (1), which activates a first burst of alarms.

If the intruder manages to break the bolts (3), the door opens and consequently the magnetic sensor, connected to the central control module (1), is activated by separating its two parts (5B) and (5A) installed in the frame ( D) and on the same door (B) and the system responds by activating new bursts of sound and/or optical alarms installed, simultaneously sending an alert via SMS to the programmed telephone number of the tenant of the dwelling, who has the option of activating the system audio and speak directly to the intruder.

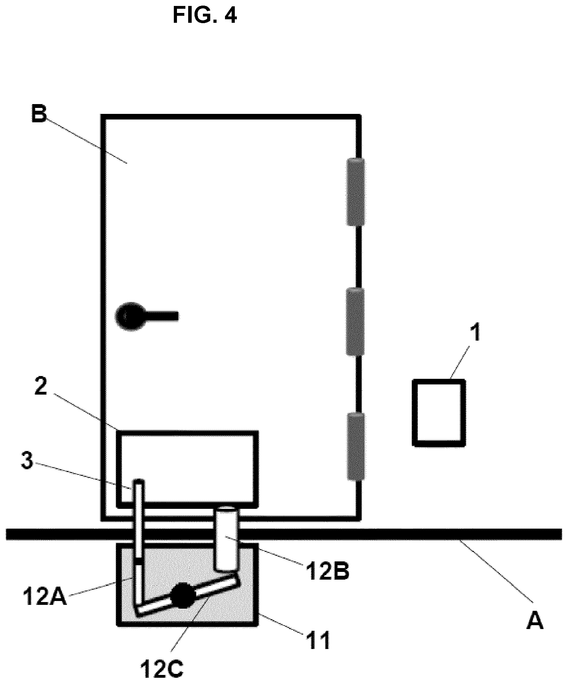

Figures 4 to 12 show different embodiments in which the arrangement of the bolts contained in the box is located in different areas in relation to the door.

Figures 4, 5 and 6 show an embodiment in which the bolt system is structured as an overlay equipment, incorporating to the basic embodiment described in the previous figures a recessed cassette or receptacle (11) in the floor (A) in order to prevent the impact of the leverage received by the bolt (3) from being transmitted to the box (2) that from this to the screws that hold it and end up impacting on the door (B).

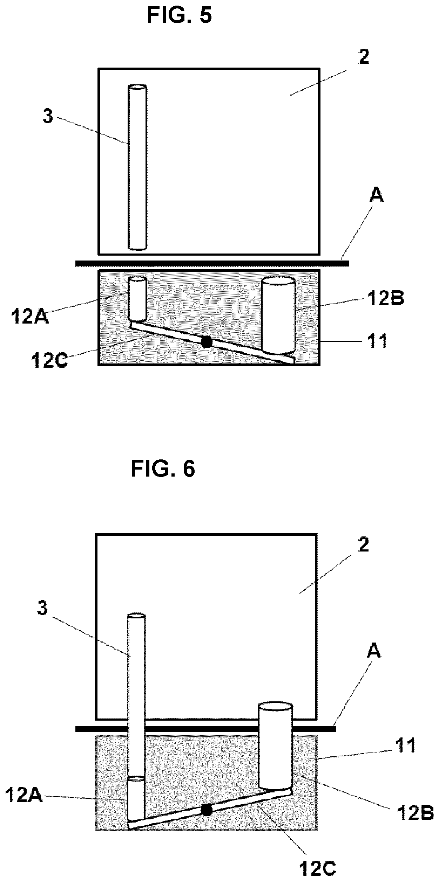

Figure 5 shows in detail how the bolt (3) is related to a lever-type mechanism integrated in a recessed cassette or receptacle (11) in the floor (A) and comprising at least three pieces (12A) (12B) (12C) that interact with each other. Figure 6 shows how, when the bolt (3) goes down, it pushes down the first moving piece (12A), which in turn acts on the second moving pieces (12C), which transmits the thrust force to the third moving piece (12B), thicker, which rises, overlapping the door (B). In this way, the impact of the leverage is cushioned when it arrives, through this third piece (12B), at the door (B).

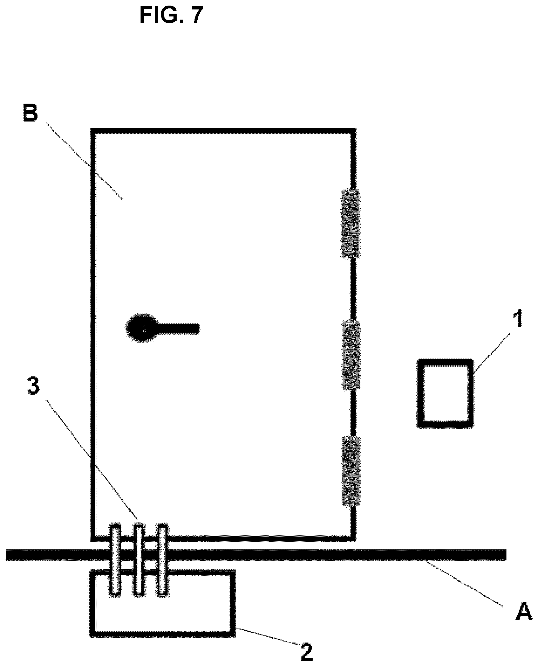

Figure 7 shows the box (2) located in the area close to the opening point of the door (B), so that the bolt (3) or bolts go up and remain behind the door (B), without having to contact with it, always inside the dwelling, adding a manual actuator related to the bolt system to unlock the equipment from inside the dwelling.

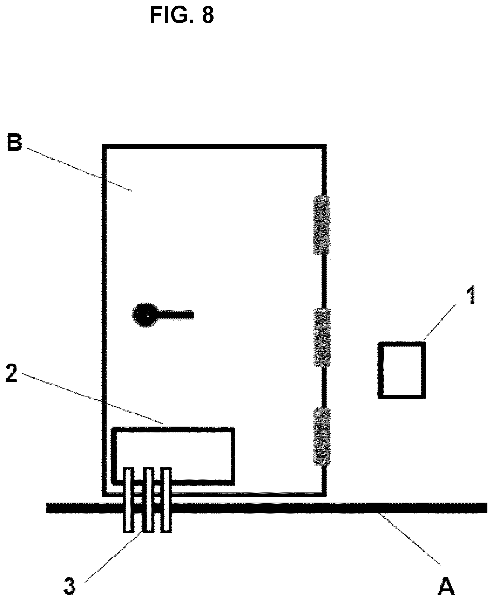

In figure 8, the bolt (3) system is presented in its technically preferred embodiment, i.e. integrated together with its activation mechanism (motor) and the vibration sensor in its box (2), which in this embodiment is clearly related to the door (B) as it is embedded in a housing previously made in it and dimensioned for this purpose. When lowering, the bolt (3) or bolts are inserted into the cavity made for this purpose in the floor (A).

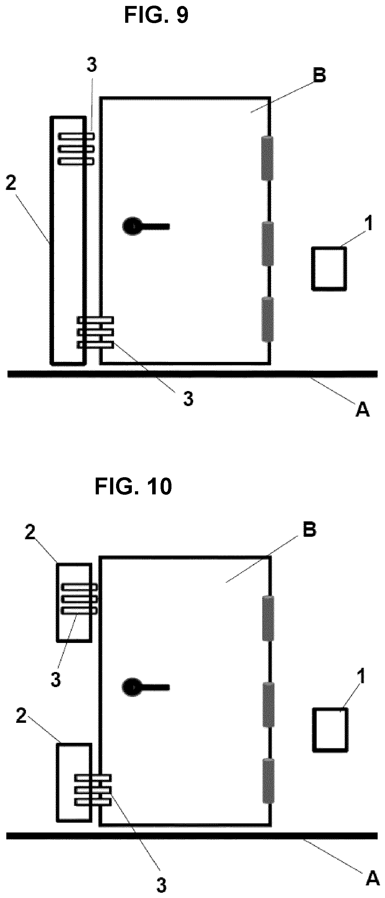

Figures 9 and 10 show two schematic views of the bolt (3) system and its activation mechanism (motor) and the vibration sensor as a locking function housed in its box (2) located in the area opposite the hinge of the door (B) using screws, either in the door frame, on the wall or on the floor. The bolt (3) or bolts always move laterally until they are blocking the opening of the door, overlapping it and without touching it at any time. These figures show embodiments with a single box (2) (figure 9) or with several boxes (2) at different height levels of the door (B) (figure 10). Also shown is the different placement of the bolts (3) when they are idle, i.e. retracted (in the case of the bolts located in the upper part), and when they are in the active position, i.e. displaced until they overlap the door (B) without touching it at any time and blocking its opening (in the case of the bolts located in the lower part).

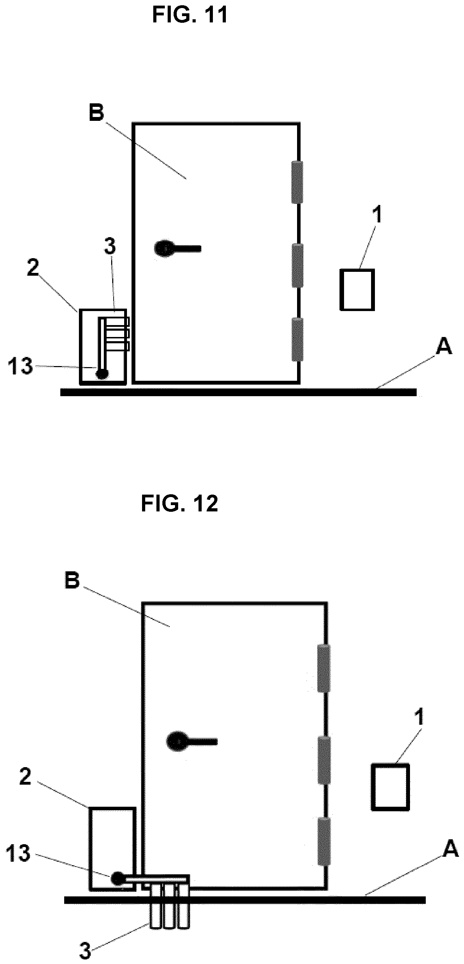

Figures 11 and 12 show the bolt system (3) and its activation mechanism (motor) and vibration sensor housed in its box (2), said bolts (3) performing an angular displacement movement. The box (2), not recessed but installed in the lower part of the door (B) frame or wall in the opposite part to the hinges, is combined with the perforations made in the floor. The bolts (3) are linked to an articulated arm (13) with a 90-degree turn towards the side of the door that drives the bolts (3), which go from an inactive horizontal position (figure 11) to an active vertical position of blocking, inserted in the cavities of the floor (A) (Figure 12).

The damping means is present either in the bolts (3), or in the box (2) or in both simultaneously in the embodiments described.

As described in these figures, it is evident that the box and consequently the bolts are located in different positions with respect to the door to adapt to different installation needs.

In these figures, the box (2) is linked to the central control module (1) by physical or, especially, wireless means, as shown in the following figures.

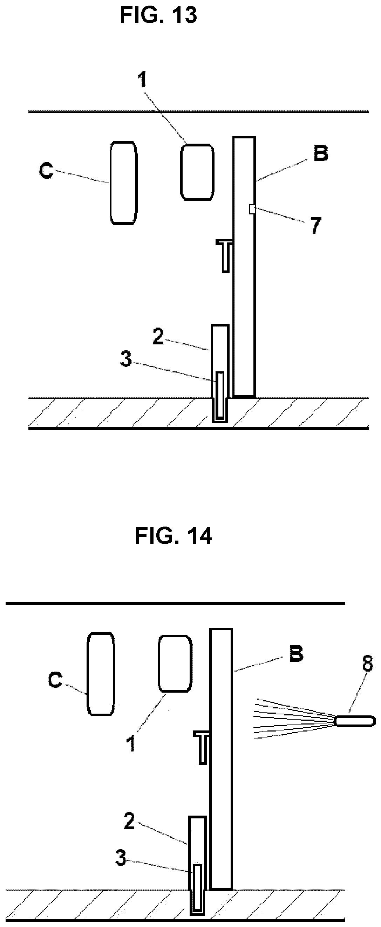

Figures 13 and 14 show schematic examples of the positioning of the electronic actuation means in two side views in which the door (B) with the box (2) with the bolts (3) it contains and the control box, the switchboard (C) of the house and the central control module (1) located inside the dwelling are visible. Figure 13 shows an example in which the means of access to the dwelling are devices that, by their very nature, require the tenant or user to access them, for example for fingerprint identification or for a module to QR code reader can interpret the graphics on a card. In this case, said devices, duly related to the corresponding electronics, are externally accessible, in this example physically located on the door located in designed and suitable areas (14) appropriate to their technical and conformation characteristics. In figure 14, the means of access to the home are wireless devices (15), for example, a Smartphone, Smartwatches or another specific control, that is, a device carried by the tenant, and act through communication technologies such as Bluetooth , GSM/GPRS, WIFI, LAN and RF (radio frequency), sending an input to which the main electronic board of the central control module (1) responds.

Based on what has been described, the advantages of this security system for dwellings doors are obvious

This system has been developed to be installed on doors with a cylinder lock with a traditional key, not to replace it but to prevent, or at least delay, the opening of the door thanks to the bolts activated by electronic means and the corresponding alarm system.

The alarm that is activated by the sensors is wired so it is immune to possible frequency inhibitors. As for the GSM signal that intervenes in the SMS notice received on the mobile phone, the claimed system is capable of incorporating means to detect if there is a frequency jammer nearby, in this case being able to activate one of the warning means already described, be it an audible or visual alarm, etc.

On the other hand, the hardened steel bolts or the double bolt with crazy rotation inside, being a physical body, can be cut with an electric tool, for example a radial grinder, but access to them must necessarily be below the door, with a reduced space of 3 mm which makes it very complicated.

The manual activation of the bolts by means of the external lever of the box ensures that people who could be inside the dwelling in the event of a total failure of the electronic system can leave it, being therefore a fundamental security element.

The hardened steel bolt or bolts, solid or hollow, may have any regular or irregular geometric shape, and obviously the cavities made in the floor will have sufficient shape and depth to house the corresponding bolt. The shape of the bolt and the cavity do not necessarily have to coincide, as long as the cavity is sufficiently dimensioned to accommodate such a bolt.

The shape of the bolt and the cavities do not affect their functionality in any way.

When the bolt is hollow, whatever its shape, the inside bolt with crazy rotation is cylindrical.

Alternatively, these bolts can also be integrated inside the door.

Optical or sound alarms are likely to respond to a smoke detector.

In short, many of the actions that occur - vibration sensor activation, audible and visual alarms, etc. - happen without necessarily the door having been breached even when the lock is forced, because the bolts continue to be a physical obstacle that blocks the opening of the door and when trying to breach them, the slightest vibration or blow will trigger the sensor or sensors of vibration. For its part, the magnetic sensor jumps when the door is opened. Because the reality is that any door can be breached simply because, as has already been mentioned, the locks are anchored and secured by low-resistance screw elements and are breached with skillful techniques, all silent. Even if it is armored and with a very complex and perfected lock, using skill techniques to force the lock or bowler or more forceful to act against the partition wall, also vulnerable. It is about having to spend as much time as possible in which the door is violated.

The claimed system can also be violated, but it forces the intruder to spend a long time on the landing of the stair, with the risk of being seen or heard by the neighbours, and if he wants to break the bolts, he needs to use electric tools that reach at least 90 decibels, which means that the noise multiplies exponentially when being on a landing of the stairs, i.e. a closed place, a fact that contradicts what the intruder usually does, enter and exit quickly and silently. Obviously, the system is functional against any attempt to force the door, whether the dwelling is empty or if it is occupied, for example at night.

The claimed system is compatible with other security elements, for example, infrared detectors, cameras, barriers, etc. to cover the dwelling if it has more accesses to it or even if it has a connection service to an alarm receiving center (ARC). It can also be incorporated as one more component to an installation that is already made in the dwelling, for example, if this installation has a switchboard, it can be connected electronically so that it works as one more peripheral, being able to send alerts instead of to the tenant, to said central and that it acts according to the protocol that it has established. Or if there is already an audible alarm, do not install another. It is about not duplicating features of the new system and another that was already installed or that is installed at another time.

The fundamental objective of the new security system for dwellings doors that is claimed is, as has been explained, to provide greater resistance to the door, considerably complicating its physical force and forcing the intruder to spend more time and to do it with more noise.

However, the dwellings have more doors and/or windows that are also access routes for intruders, for example in interior to yards, terraces and balconies. These accesses may have protection bars that are vulnerable to mechanical tools such as picks or shears.

Given the nature of these exterior doors, the security system that is presented is modified in some of its characteristics since it would be easy to cut the wiring or lift the bolts by means of manual actuation with a lever in the box. For this reason, a second model of the system is proposed, in which the installation lacks screws and the box lacks a lever for manual opening, being totally watertight as it is welded and includes a rechargeable power battery through a connector that will be connected to carry out the charge of said battery and will be disconnected once the charge has been made. In the event of a breakdown, the technical staff must use electrical machinery.

Obviously, this second version of the system carries some risk of being blocked and requires more technical intervention to open it, but as it is a secondary access to the dwelling, the tenant continues to use the main access, while the possible intruder, even if he forces the bowler, you will find a physically strong and strong system.

This interactive security and locking system implement a plurality of means, both physical and wireless, to enable not only the opening of the door but also peripheral elements of dwelling security.

The bolt can adopt other conformations that fulfill the same function, i.e. that have mobility in response to the activation of the motors and that either inserted into the cavities made in the floor or remain a few millimeters from the door, in its inner part, so that in any case they act as effective means of blocking.

In the embodiment in which the box with the bolt system is embedded in a housing previously made in the door, it is obvious that this technical solution requires a commercial relationship with door manufacturers since it must be manufactured with such a housing provided with a cover.

With the different arrangements of the bolt system described, it is achieved that the attempt to force the door implies the use of unusual tools, for example machinery emitting more than 90dB, and requires a long time, facts that tend to make the intruder give up.

At the same time, said system requires that the user, tenant of the dwelling, have the maximum guarantees to access their home, and that is why the wide variety of means detailed in this invention is required. Obviously not all the means that are contemplated must be present together in the interactive system that is claimed. Depending on each door, the demand for the level of security required and especially the need and/or capacity of each tenant, the choice of one or another means or the implementation of several of them. This assumption derives, for example, from the fact that not all people have a Smartphone or do not trust non-tangible means of opening.

In any case, each and every one of the aforementioned means, alone or in combination with each other, allow access to the dwelling, controlling the movement of the bolts in an upward direction - the door opens - or downward - the door remains closed and locked, ensuring the maximum inviolability of the dwelling and at the same time guaranteeing its opening by its tenant.

From what is described in these pages, it is evident that the tenant always has an operational resource that allows the door to be opened in the event of failure or violation of the installed means of physical access, for example failure of fingerprint recognition or alphanumeric keyboard, having various support technologies such as Bluetooth, SMS, etc. Likewise, as an optional opening device, the tenant can have a secondary electronic board located in the box attached to the door or in the central control module, if he chooses to install it.

The interactive system, as a self-contained system, is capable to have a connection service to an alarm reception center (ARC), so that the various sensors it incorporates send and contact the aforementioned ARC, which activates the corresponding protocols. In a second embodiment, the interactive system acts as a peripheral element of an alarm center, i.e. a local electronic switchboard installed in the dwelling, limiting its electronic content and therefore reducing its functions.

It is not considered necessary to make this description more extensive, so that any person skilled in the art understands the scope of the invention and the advantages derived from it. The materials, shape, size, position, direction and angle will be subject to variation as long as this does not imply an alteration in the essence of the invention. The terms in which this report has been written must always be taken in a broad and non-limiting sense.

## Claims

1. st Interactive security and locking system for dwellings doors, of the type that are related to the floor (A), the door (B) and the electrical switchboard (C) of the dwelling, **characterized in that** it comprises mechanical means of physical blocking, in particular at least one bolt (3) and electrical and electronic security means for its opening and closing, and means for its management through an application (App) for Android and IOS systems on Smartphone and Smartwatch mobile devices, connecting said App with the security lock directly (locally) through Bluetooth and GSM and remotely (external server) through Wi-Fi, GPRS and LAN, also implementing means of access control to the home, means for connection auxiliary peripheral security elements and a security device for opening the door, the system itself acting as a self-contained system or as a peripheral unit.

2. nd Interactive security and locking system for dwellings doors, according to claim 1, **characterized in that** the mechanical physical locking means also comprise main and secondary motors (7) and at least one vibration sensor (6), all located together with the bolts (3) in a box (2) connected by wiring with a central control module (1).

3. rd Interactive security and locking system for dwellings doors, according to claims 1 and 2, **characterized in that** the electrical and electronic means are located in the central control module (1).

4. th Interactive security and locking system for dwellings doors, to claims 1 and 2, **characterized in that** the bolts (3) are mobile and travel upwards and downwards.

5. th Interactive security and locking system for home doors, according to claims 1 and 2, **characterized in that** the bolts (3) are activated by a transmitter/receiver control via radio with an encrypted signal and/or evolutionary code received by the main motor (7) or, if applicable, the secondary one.

6. th Interactive security and locking system for dwellings doors, according to claim 2, **characterized in that** the vibration sensor (6), connected to the central control module (1) is close to the bolts (3), both anchored in the same support.

7. th Interactive security and locking system for dwellings doors, according to the claims 1 and 2, **characterized in that** the mechanical physical locking means include a magnetic sensor consisting of two pieces located in parallel and close to contact each other, one piece (5B) anchored on the frame (D) and the other (5A) on the door (B), this magnetic sensor being connected to the central control module (1).

8. th Interactive security and locking system for dwellings doors, according to claim 2, **characterized in that** on the floor (A) of the dwelling, coinciding with the position of the bolts (3) housed in the box (2), there are two cavities (4) suitable for housing the lower part of said bolts (3) up to the floor when the door (B) is closed.

9. th Interactive security and locking system for dwellings doors, according to claim 2, **characterized in that** when the door (B) is open, the bolts (3) are retracted or raised and located completely inside the box (2), the two magnetic sensor parts (5B) and (5A) being simultaneously separated.

10. th Interactive security and locking system for dwellings doors, according to claim 2, **characterized in that** the bolts (3) as a whole are linked at their upper end to a longitudinal bar (10) that drags them upwards by the action of a lever (9) protruding from the outside of the box (2), which internally incorporates a rail (8) on which said lever (9) seated and which regulates its travel.

11. th Interactive security and locking system for dwellings doors, according to claim 1, **characterized in that** the bolts (3) are made of tempered and solid steel, of any plan or regular or irregular geometric shape.

12. th Interactive security and locking system for dwellings doors, according to claim 1, **characterized in that** the bolts (3) are hollow, housing a second crazy-turning cylindrical bolt inside.

13. th Interactive security and locking system for dwellings doors, according to claim 8 and 11, **characterized in that** each cavity (4) made in the floor (A) has a shape and depth sufficient to house the corresponding bolt.

14. th Interactive security and locking system for dwellings doors, according to claim 1, **characterized in that** the bolts (3) are integrated inside the door (B).

15. th Interactive security and locking system for dwellings doors, according to claim 2, **characterized in that** the secondary motor (7) is activated via GSM by means of an SMS.

16. th Interactive security and locking system for dwellings doors, according to claim 2, **characterized in that** the secondary motor (7) is activated via a wireless switch by means of a wireless transmitter.

17. th Interactive security and locking system for dwellings doors, according to claim 1, **characterized in that** the electrical and electronic means activate a sound and/or optical alarm system.

18. th Interactive security and locking system for dwellings doors, according to claims 6, 7 and 17, **characterized in that** when at least one of the sensors is activated, the electronic system activates at least one interior siren of at least 90 decibels.

19. th Interactive security and locking system for dwellings doors, according to claims 6, 7 and 17, **characterized in that** when at least one of the sensors is activated, the electronic system through technology GSM an SMS to previously registered phones.

20. th Interactive security and locking system for dwellings doors, according to claim 17, **characterized in that** the alarm system responds to a smoke detector.

21. st Interactive security and locking system for dwellings doors, according to claim 17, **characterized in that** the alarm system incorporates a loudspeaker.

22. nd Interactive locking and security system for dwellings doors, according to claims 1 and 2, **characterized in that** the bolts (3) and/or the box (2) incorporate a damping means to mitigate the impact of leverage on the box ( 2) and on the door (B), placing the box (2) and consequently the bolts (3) in different positions in relation to the aforementioned door (B) to act as a physical means of locking and unlocking its movement (B).

23. rd Interactive locking and security system for dwellings doors, according to claim 22, **characterized in that** the damping element of the bolts (3) is an intermediate piece that makes them flexible.

24. th Interactive security and locking system for dwellings doors, according to claims 2 and 22, **characterized in that** the bolt (3), integrated in the box (2), with a lever-type mechanism integrated in a recessed cassette or receptacle (11) in the floor (A), comprises at least three pieces (12A) (12B) (12C) that interact with each other, the first (12A) of which receives the impact of the bolt (3) when it lowers, acting on the second moving piece (12C) that transmits the thrust force to the third moving piece (12 B), which rises, overlapping the door (B).

25. th Interactive security and locking system for dwellings doors, according to claim 2, **characterized in that** the box (2), housing the bolt (3) and its consequent activation mechanism (motor) and the vibration sensor, is located in the area close to the door opening point (B), so that the bolt (3) or bolts go up and remain behind the door (B), without no contact with it.

26. th Interactive security and locking system for dwellings doors, according to claim 25, **characterized in that** it has a manual actuator to unlock the equipment.

27. th Interactive security and locking system for dwellings doors, according to claim 2, **characterized in that** the box (2), housing the bolt (3) and its consequent activation mechanism (motor) and the vibration sensor, it is embedded in a housing previously made in the door (B), so that the bolt (3) or bolts, when lowered, are inserted into a cavity made for this purpose in the floor (A).

28. th Interactive security and locking system for dwellings doors, according to claim 2, **characterized in that** the box (2), housing the bolt (3) and its consequent activation mechanism (motor) and the vibration sensor inside it , is located in one or more areas opposite the door (B) hinge by means of screws, moving the bolt (3) or bolts laterally from a retracted position, inactive, to an active position, displaced until they overlap the door ( B) without touching it at any time.

29. th Interactive security and locking system for dwellings doors, according to claim 2, **characterized in that** the box (2), housing the bolt (3) and its consequent activation mechanism (motor) and the vibration sensor, non-recessed, is located on the side of the lower part of the door (B) frame or wall opposite the hinge, said bolts (3) performing an angular displacement movement as they are linked with an articulated arm (13) with a rotation of 90 degrees towards the side of the door (B), dragging the bolts (3), which go from an inactive horizontal position to an active vertical locking position, inserted into the cavities in the floor (A).

30. th Interactive security and locking system for dwellings doors, according to claims 2 and 7, **characterized in that** the central control module (1) and the magnetic sensor-are integrated in the box (2) that contains the bolts, activation mechanism (motor) and vibration sensor.

31. st Interactive security and locking system for dwellings doors, according to claim 1, **characterized in that** the connection of the means of access control to the dwelling and of the means for the connection of peripheral elements is carried out through wireless technology, being linked to a device carried by the tenant, acting through communication technologies such as Bluetooth, GSM/GPRS, WIFI and RF (radio frequency) that send an input to which the main electronic board of the central control module (1) responds.

32. nd Interactive security and locking system for dwellings doors, according to claim 1, **characterized in that** the connection of the means of control of access to the dwelling and of the means for the connection of peripheral elements is carried out using LAN technology.

33. rd Interactive security and locking system for dwellings doors, according to claim 1, **characterized in that** the access control means and the peripheral elements are externally accessible and duly related to the corresponding electronics.

34. th Interactive security and locking system for dwellings doors, according to claim 1, **characterized in that** the access control means are devices that act as switches that activate and deactivate the equipment connected to the main electronic board housed in the central control module (1) that governs the operation of the system.

35. th Interactive security and locking system for dwellings doors, according to claim 34, **characterized in that** the access control devices comprise an alphanumeric keyboard.

36. th Interactive security and locking system for dwellings doors, according to claim 34, **characterized in that** the access control devices comprise a flat magnetic key.

37. th Interactive security and locking system for dwellings doors, according to claim 34, **characterized in that** the access control devices comprise a tubular key lock.

38. th Interactive security and locking system for dwellings doors, according to claim 34, **characterized in that** the access control devices comprise a manual push button.

39. th Interactive security and locking system for dwellings doors, according to claim 34, **characterized in that** the access control devices comprise a manual IR infrared push button.

40. th Interactive security and locking system for dwellings doors, according to claim 34, **characterized in that** the access control devices comprise an RF emitter (radio frequency).

41. st Interactive security and locking system for dwellings doors, according to claim 1 and 34, **characterized in that** the access control devices comprise a technological module suitable for being read by a mobile electronic device through the APP.

42. nd Interactive security and locking system for dwellings doors, according to claim 41, **characterized in that** the technological module is an integrated circuit of low frequency (LF: 125 - 134 kHz and 140 - 148.5 kHz) and high frequency (HF: 13.56 MHz) RFID wireless technology, RFID, and its corresponding reader.

43. rd Interactive security and locking system for dwellings doors, according to claim 41, **characterized in that** the technology module is a CQR code reader.

44. th Interactive security and locking system for dwellings doors, according to claim 41, **characterized in that** the technological module is an NFC-type short-range wireless technology integrated circuit and a reader thereof.

45. th Interactive security and locking system for dwellings doors, according to claim 34, **characterized in that** the access control devices comprise a biometric fingerprint sensor type reader.

46. th Interactive security and locking system for dwellings doors, according to claim 34, **characterized in that** the access control devices comprise a biometric facial sensor type reader.

47. th Interactive security and locking system for dwellings doors, according to claim 34, **characterized in that** the access control devices comprise a biometric of the palm fingerprint sensor type reader.

48. th Interactive security and locking system for dwellings doors, according to claim 34, **characterized in that** the access control devices comprise a biometric of the eye iris sensor type reader.

49. th Interactive security and locking system for dwellings doors, according to claim 34, **characterized in that** the access control devices comprise a biometric forearm vein sensor type reader.

50. th Interactive security and locking system for dwellings doors, according to claim 34, **characterized in that** the access control devices comprise a voice identifier device.

51. st Interactive security and locking system for dwellings doors, according to claim 1, **characterized in that** the peripheral auxiliary security elements comprise an IP camera.

52. nd Interactive security and locking system for dwellings doors, according to claim 1, **characterized in that** the auxiliary peripheral security elements comprise a volumetric.

53. rd Interactive security and locking system for dwellings doors, according to claim 1, **characterized in that** the peripheral auxiliary security elements comprise an access control camera.

54. th Interactive security and locking system for dwellings doors, according to claim 1, **characterized in that** the peripheral auxiliary safety elements comprise an infrared movement sensor.

55. th Interactive security and locking system for dwellings doors, according to claim 1, **characterized in that** the peripheral auxiliary security elements comprise a radar movement sensor.

56. th Interactive security and locking system for dwellings doors, according to claim 1, **characterized in that** the peripheral auxiliary security elements comprise a camera movement sensor.

57. th Interactive security and locking system for dwellings doors, according to claim 1, **characterized in that** the peripheral auxiliary security elements comprise a microwave movement sensor.

58. th Interactive security and locking system for dwellings doors, according to claim 1, **characterized in that** the auxiliary safety auxiliary peripheral elements comprise a thermal sensor.

59. th Interactive security and locking system for dwellings doors, according to claim 1, **characterized in that** the auxiliary peripheral security elements comprise a thermographic camera.

60. th Interactive security and locking system for dwellings doors, according to claim 1, **characterized in that** the peripheral auxiliary safety elements comprise an optical and/or sound alert terminal.

61. st Interactive security and locking system for dwellings doors, according to claim 1, **characterized in that** the peripheral auxiliary safety elements comprise a gas sensor.

62. nd Interactive security and locking system for dwellings doors, according to claim 1, **characterized in that** the peripheral auxiliary security elements comprise a humidity sensor.

63. rd Interactive security and locking system for dwellings doors, according to claim 1, **characterized in that** the peripheral auxiliary safety elements comprise a smoke sensor.

64. th Interactive security and locking system for dwellings doors, according to claim 1, **characterized in that** the peripheral auxiliary safety elements comprise a vibration sensor.

65. th Interactive security and locking system for dwellings doors, according to claim 1, **characterized in that** the peripheral auxiliary security elements comprise a magnetic sensor.

66. th Interactive security and locking system for dwellings doors, according to claim 1, **characterized in that** the safety device for opening the door is specified in a secondary electronic board.

67. th Interactive security and locking system for dwellings doors, according to claim 66, **characterized in that** the secondary electronic board is housed in the central control module (1).

68. th Interactive security and locking system for dwellings doors, according to claim 66, **characterized in that** the secondary electronic board is housed in the box (2).

69. th Interactive security and locking system for dwellings doors, according to claim 1, **characterized in that**, as an autonomous system, it has a connection service to an alarm reception center (ARC).

70. th Interactive security and locking system for dwellings doors, according to claim 1, **characterized in that** as a peripheral unit it is linked to a local electronic switchboard installed in the dwelling.
